# EUROPÄISCHE PATENTSCHRIFT

(11) **EP 0 771 788 B1**
(45) Veröffentlichungstag und Bekanntmachung des Hinweises auf die Patenterteilung: **12.01.2000**
(21) Anmeldenummer: 96116938.0
(22) Anmeldetag: 22.10.1996
(51) Int. Cl.: C07C 381/10, A61K 31/165

(54) **Substituierte Sulfonimidamide, Verfahren zu ihrer Herstellung, ihre Verwendung als Medikament oder Diagnostikum sowie sie enthaltendes Medikament**
Substituted sulphonimidamides, method for their preparation, their use as medicament or diagnostic and medicament containing them
Sulfonimidamides substitués, procédé pour leur préparation, leur utilisation comme médicament ou agent diagnostique, ainsi que médicament les contenant

(30) Priorität: 03.11.1995 DE 19540995
(43) Veröffentlichungstag der Anmeldung: 07.05.1997
(73) Patentinhaber: HOECHST AKTIENGESELLSCHAFT, 65929 Frankfurt am Main (DE)
(72) Erfinder: Kleemann, Heinz-Werner, Dr., 65474 Bischofsheim (DE); Brendel, Joachim, Dr., 61118 Bad Vilbel (DE); Schwark, Jan-Robert, Dr., 65929 Frankfurt (DE); Weichert, Andreas, Dr., 63329 Egelsbach (DE); Lang, Hans Jochen, Dr., 65719 Hofheim (DE); Albus, Udo, Dr., 61197 Florstadt (DE); Scholz, Wolfgang, Dr., 65760 Eschborn (DE)

(56) Entgegenhaltungen:
- EP-A- 0 556 674
- US-A- 5 091 394

## Beschreibung

Die Erfindung betrifft Sulfonimidamide der Formel I 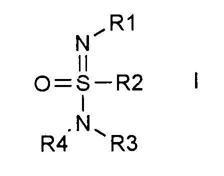 worin bedeuten:
mindestens einer der drei Substituenten R(1), R(2) und R(3)
   ein Benzoylguanidin, 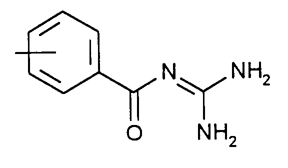
      das im Phenylteil unsubstituiert oder substituiert ist mit 1 - 4 Resten ausgewählt aus der Gruppe bestehend aus Alkyl mit 1, 2, 3, 4, 5, 6, 7 oder 8 C-Atomen, Alkenyl mit 2, 3, 4, 5, 6, 7 oder 8 C-Atomen, -(CH₂)ₘ-R(14), F, Cl, Br, I, -C≡N, CF₃, R(22)SO₂-, R(23)R(24)N-CO-, R(25)-CO-, R(26)R(27)N-SO₂, -OR(35), -SR(35) oder -NR(35)R(36);
      m Null, 1 oder 2;
      R(14)
         -(C₃-C₈)-Cycloalkyl oder Phenyl,
            welches nicht substituiert oder substituiert ist mit 1 - 3 Substituenten ausgewählt aus der Gruppe bestehend aus F und Cl, -CF₃, Methyl, Methoxy und -NR(15)R(16);
            R(15) und R(16)
               unabhängig voneinander Wasserstoff oder - CH₃;
      R(22), R(23), R(25) und R(26)
         unabhängig voneinander Alkyl mit 1, 2, 3, 4, 5, 6, 7 oder 8 C-Atomen, Alkenyl mit 2, 3, 4, 5, 6, 7 oder 8 C-Atomen, (CH₂)ₙR(29) oder -CF₃;
         n Null, 1, 2, 3 oder 4;
         R(29) -(C₃-C₇)-Cycloalkyl oder Phenyl,
            welches nicht substituiert ist oder substituiert mit 1 - 3 Substituenten ausgewählt aus der Gruppe bestehend aus F, Cl, -CF₃, Methyl, Methoxy und -NR(30)R(31);
            R(30) und R(31)
               Wasserstoff oder Alkyl mit 1, 2, 3 oder 4 C-Atomen;
         oder
      R(23), R(25) und R(26)
         Wasserstoff;
      R(24) und R(27)
         unabhängig voneinander Wasserstoff oder Alkyl mit 1, 2, 3 oder 4 C-Atomen;
         oder
      R(23) und R(24) sowie R(26) und R(27)
         gemeinsam 5 oder 6 Methylengruppen, von denen eine CH₂-Gruppe durch Sauerstoff, -S-, -NH-, -NCH₃ oder - N-Benzyl ersetzt sein kann;
      R(35) und R(36)
         unabhängig voneinander Wasserstoff oder Alkyl mit 1, 2, 3, 4, 5 oder 6 C-Atomen;
         oder
      R(35) und R(36)
         gemeinsam 4 - 7 Methylengruppen, von denen eine CH₂-Gruppe durch Sauerstoff, -S-, -NH-, -NCH₃ oder - N-Benzyl ersetzt sein kann;
         oder
      R(35)
         Phenyl,
            welches nicht substituiert ist oder substituiert mit 1 - 3 Substituenten ausgewählt aus der Gruppe bestehend aus F, Cl, -CF₃, Methyl, Methoxy, SO₂R(5), SO₂NR(6)R(7) und -NR(32)R(33);
            R(5) Alkyl mit 1, 2, 3, 4, 5 oder 6 C-Atomen
            R(6) und R(7)
               unabhängig voneinander Wasserstoff oder Alkyl mit 1, 2, 3 oder 4 C-Atomen;
            R(32) und R(33)
               unabhängig voneinander Wasserstoff oder Alkyl mit 1, 2, 3 oder 4 C-Atomen;
         oder
      R(35)
         C₁-C₉-Heteroaryl,
            das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
und die jeweils anderen Substituenten R(1), R(2) und R(3)
   unabhängig voneinander Alkyl mit 1, 2, 3, 4, 5, 6, 7 oder 8 C-Atomen, (CH₂)ₚR(10)
   p Null, 1, 2, 3 oder 4;
   R(10) Phenyl,
      welches nicht substituiert ist oder substituiert mit 1 - 3 Substituenten ausgewählt aus der Gruppe bestehend aus F, Cl, -CF₃, Methyl, Methoxy, -SO₂NR(17)R(8) und -SO₂R(9);
      R(17) und R(8)
         unabhängig voneinander Wasserstoff oder Alkyl mit 1, 2, 3 oder 4 C-Atomen;
      R(9) Alkyl mit 1, 2, 3 oder 4 C-Atomen;
oder der jeweils andere Rest R(1) und R(3)
   Wasserstoff,
R(4) Wasserstoff oder Alkyl mit 1, 2, 3 oder 4 C-Atomen;
   sowie deren pharmazeutisch verträgliche Salze.

Bevorzugt sind Verbindungen der Formel I, in denen bedeuten:
einer der drei Substituenten R(1), R(2) und R(3) ein Benzoylguanidin, 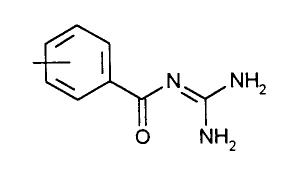
   das im Phenylteil unsubstituiert oder substituiert ist mit 1 - 3 Substituenten ausgewählt aus der Gruppe bestehend aus Alkyl mit 1, 2, 3 oder 4 C-Atomen, Alkenyl mit 2, 3 oder 4 C-Atomen, -(CH₂)ₘR(14), F, Cl, CF₃, R(22)SO₂-, R(23)R(24)N-CO-, R(25)-CO-, R(26)R(27)N-SO₂, -OR(35), -SR(35) und -NR(35)R(36);
   R(22) und R(25)
      unabhängig voneinander Methyl oder -CF₃;
   R(23), R(24), R(26) und R(27)
      unabhängig voneinander Wasserstoff oder Methyl;
   m Null, 1 oder 2;
      R(14) -(C₃-C₆)-Cycloalkyl oder Phenyl,
         welches nicht substituiert oder substituiert ist mit 1 - 2 Substituenten ausgewählt aus der Gruppe bestehend aus F und Cl, -CF₃, Methyl und Methoxy
   R(35) und R(36)
      unabhängig voneinander Wasserstoff oder Alkyl mit 1, 2, 3 oder 4 C-Atomen;
      oder
   R(35) und R(36)
      gemeinsam 4 - 6 Methylengruppen, von denen eine CH₂-Gruppe durch Sauerstoff, -S-, -NH- oder -NCH₃ ersetzt sein kann;
      oder
   R(35)
      Phenyl,
         welches nicht substituiert ist oder substituiert mit 1 - 2 Substituenten ausgewählt aus der Gruppe bestehend aus F, Cl, -CF₃, Methyl, Methoxy, SO₂R(5) und SO₂NR(6)R(7)
         R(5)
            Alkyl mit 1, 2, 3 oder 4 C-Atomen
         R(6) und R(7)
            unabhängig voneinander Wasserstoff , Methyl oder Ethyl;
         oder
      R(35)
         C₁-C₉-Heteroaryl,
            das unsubstituiert oder substituiert ist mit 1 - 2 Substituenten ausgewählt aus der Gruppe bestehend aus F, Cl, CF₃, CH₃ und Methoxy;
und die jeweils anderen Substituenten R(1), R(2) und R(3)
   unabhängig voneinander Alkyl mit 1, 2, 3, 4, 5 oder 6 C-Atomen oder (CH₂)ₚR(10)
   p Null, 1 oder 2;
   R(10) Phenyl,
      welches nicht substituiert ist oder substituiert mit 1 - 2 Substituenten ausgewählt aus der Gruppe bestehend aus F, Cl, CF₃, Methyl, Methoxy, -SO₂NR(17)R(8) und -SO₂R(9);
      R(17) und R(8)
         unabhängig voneinander Wasserstoff, Methyl oder Ethyl;
      R(9) Methyl oder Ethyl;
oder der jeweils andere Substituent R(1) und R(3)
   Wasserstoff,
R(4)
   Wasserstoff oder Alkyl mit 1, 2, 3 oder 4 C-Atomen;
   sowie deren pharmazeutisch verträgliche Salze.

Besonders bevorzugt sind Verbindungen der Formel I, in denen bedeuten:
einer der Substituenten R(1), R(2) und R(3) ein Benzoylguanidin, 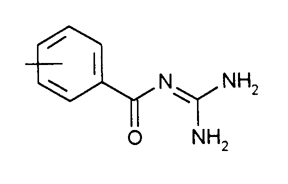
   das im Phenylteil unsubstituiert oder substituiert ist mit 1 - 2 Resten ausgewählt aus der Gruppe bestehend aus Alkyl mit 1, 2, 3 oder 4 C-Atomen, R(14), F, Cl, CF₃, R(22)SO₂-, R(23)R(24)N-CO-, R(25)-CO-, R(26)R(27)N-SO₂, -OR(35), -SR(35) und -NR(35)R(36);
   R(22) und R(25)
      unabhängig voneinander Methyl oder CF₃;
   R(23), R(24), R(26) und R(27)
      unabhängig voneinander Wasserstoff oder Methyl;
   R(14) -(C₃-C₆)-Cycloalkyl oder Phenyl,
      welches nicht substituiert oder substituiert ist mit einem Substituenten ausgewählt aus der Gruppe bestehend aus F und Cl, -CF₃, Methyl und Methoxy;
   R(35) und R(36)
      unabhängig voneinander Wasserstoff oder Alkyl mit 1, 2, 3 oder 4 C-Atomen;
      oder
   R(35)
      Phenyl,
         welches nicht substituiert oder substituiert ist mit einem Substituenten ausgewählt aus der Gruppe bestehend aus F, Cl, -CF₃, Methyl, Methoxy, SO₂CH₃ und SO₂NR(6)R(7);
         R(6) und R(7)
            unabhängig voneinander Wasserstoff, Methyl oder Ethyl;
      oder
   R(35)
      C₁-C₉-Heteroaryl,
         das unsubstituiert oder substituiert ist mit einem Substituenten ausgewählt aus der Gruppe bestehend aus F, Cl, CF₃, CH₃ und Methoxy;
und die jeweils anderen Substituenten R(1), R(2) und R(3)
   unabhängig voneinander Alkyl mit 1, 2, 3 oder 4 C-Atomen oder Phenyl,
      welches nicht substituiert ist oder substituiert mit einem Substituenten ausgewählt aus der Gruppe bestehend aus F, Cl, CF₃, Methyl, Methoxy, -SO₂NR(17)R(8) und -SO₂CH₃;
      R(17) und R(8)
         unabhängig voneinander Wasserstoff, Methyl oder Ethyl;
   oder
R(1) Wasserstoff oder Alkyl mit 1, 2, 3 oder 4 C-Atomen;
oder R(3)
   Wasserstoff;
R(4) Wasserstoff oder Alkyl mit 1, 2, 3 oder 4 C-Atomen;
   sowie deren pharmazeutisch verträgliche Salze.

Die bezeichneten Alkylreste können sowohl geradkettig wie verzweigt vorliegen.

Unter (C₁-C₉)-Heteroaryl werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(4) ein oder mehrere Asymmetriezentren, so können diese unabhängig voneinander sowohl S als auch R konfiguriert sein. Die Verbindungen können als optische Isomere, als Diastereomere, als Racemate oder als Gemische derselben vorliegen.

Die bezeichneten Alkylreste können sowohl geradkettig als auch verzweigt vorliegen.

Die Erfindung betrifft weiterhin ein Verfahren zur Herstellung der Verbindungen I, dadurch gekennzeichnet, daß man Verbindungen der Formel II

HS-R(2)' II

worin R(2)' für einen gegebenenfalls geeignet geschützten Rest R(2) steht, mit einem nach allgemein bekannten Verfahren in situ hergestellten Monochlor- oder Monobromamin umsetzt. Diese Umsetzung erfolgt in einem inerten Lösemittel wie CH₂Cl₂, CHCl₃ oder CCl₄ bei einer Temperatur zwischen RT und dem Schmelzpunkt der verwendeten Lösung, bevorzugt zwischen 0°C und -40°C. In einigen Fällen kann auch das dem Haloamin zugrundeliegende Amin oder auch Wasser als Lösemittel eingesetzt werden. Man erhält Sulfonimidamide der Formel III, 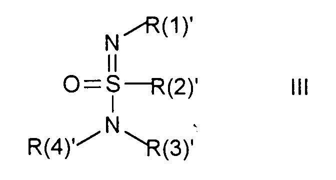 worin R(1)', R(2)' R(3)' und R(4)' für einen gegebenenfalls geeignet geschützten oder vorläufigen Rest R(1), R(2) R(3) und R(4) steht. Nachträgliche Einführung eines Restes R(3) kann über aromatische nucleophile Substitutionen erfolgen, die für die den Sulfonimidamiden der Formel III analogen Sulfonamiden allgemein bekannt sind.

Die Guanylierung der Benzoesäurefunktion erfolgt nach prinzipiell bekannten Methoden, indem eine leicht nucleophil abspaltbare Fluchtgruppe L in der Verbindung IV, in welcher mindestens einer der Substituenten R(1'*'*), R(2'*'*) oder R(3'*'*) wie R(1), R(2) und R(3) definiert sind, jedoch statt der Guanidin-Gruppe eine leicht abspaltbare Fluchtgruppe L trägt, 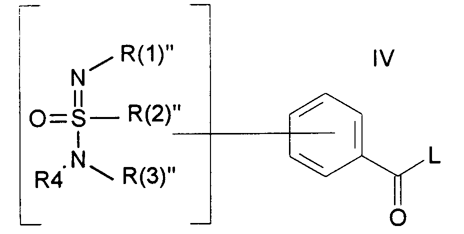 diese leicht nucleophil abspaltbare Gruppe durch Guanidin substituiert.

Die Einführung der im Phenylteil mit Schwefel-, Sauerstoff- oder Stickstoffnucleophilen substituierten Benzoesäure-Derivate gelingt durch literaturbekannte Methoden der nucleophilen Substitution am Aromaten. Als Abgangsgruppe am Benzoesäurederivat haben sich bei dieser Substitution Halogenide und Trifluormethansulfonate bewährt. Man arbeitet vorteilhaft in einem dipolar aprotischen Lösungsmittel, wie DMF oder TMU, bei einer Temperatur von 0°C bis zum Siedepunkt des Lösungsmittels, bevorzugt von 80°C bis zum Siedepunkt des Lösungsmittels. Als Säurefänger dient vorteilhaft ein Alkali- oder Erdalkalisalz mit einem Anion hoher Basizität und geringer Nucleophilie, zum Beispiel K₂CO₃ oder CsCO₃.

Funktionelle Gruppen in den Resten R(1) bis R(4), die als Reduktionsmittel fungieren können, werden vorteilhaft nach der Einführung der Sulfonimidamid-Gruppe aufgebaut. Die Ansprüche an die Schutzgruppen während der Herstellung der Sulfonimidamid-Funktionalität leiten sich aus dem Reaktions-Typ ab. Es handelt sich dabei um eine Oxidation mit einem starken Oxidationsmittel. Stickstoffhaltige Heteromaten, wie zum Beispiel Pyridinyl-Gruppen, können in Form der entsprechenden N-Oxide geschützt werden.

Die Oxidation des Stickstoffs im Heteroaryl-Substituenten gelingt an geeigneten Zwischenverbindungen wie dem Benzoesäureester mit im Prinzip bekannten Methoden. Bewährt hat sich beispielsweise m-Chlorperbenzoesäure in einem inerten Lösungsmittel wie Methylenchlorid bei einer Temperatur zwischen -30^{o}C und dem Siedepunkt des Lösungsmittels. Methoden zur Reduktion von Heteroaryl-N-Oxiden wie zum Beispiel Pyridin-N-Oxiden sind ebenfalls im Prinzip bekannt. Bewährt hat sich beispielsweise die Umsetzung mit Triphenylphosphin oder Trimethylphosphit (C. Kaneko, A. Yamamoto, M. Gomi Heterocycles 12, 227 (1979)).

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Sulfonimidamide I sind im allgemeinen schwache Basen und können Säure unter Bildung von Salzen binden. Als Säureadditionssalze kommen Salze aller pharmakologisch verträglichen Säuren in Frage, beispielsweise Halogenide, insbesondere Hydrochloride, Ascorbate, Lactate, Sulfate, Citrate, Tartrate, Acetate, Phosphate, Methylsulfonate, p-Toluolsulfonate.

In der US-Patentschrift 5 091 394 (HOE 89/F 288) und in der europäischen Offenlegungsschrift 0 556 674 (HOE 92/F 034) werden Benzoylguanidine beschrieben, in welchen aber die Substituenten nicht die nach der vorliegenden Erfindung beanspruchten Bedeutungen haben. Es sind keine Benzolsulfonimidamid-Derivate beschrieben. Außerdem läßt die Wasserlöslichkeit dieser bekannten Benzoylguanidine zu wünschen übrig.

Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, Insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den meisten bekannten Verbindungen weisen die Verbindungen nach der Erfindung eine signifikant verbesserte Wasserlöslichkeit auf. Daher sind sie wesentlich besser für i.v.-Applikation geeignet.

Die erfindungsgemäßen Verbindungen zeichnen sich gegenüber den bekannten gut wasserlöslichen Verbindungen durch ihre ausgezeichnete Bioverfügbarkeit und Pharmakokinetik aus.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg Körpergewicht, vorzugsweise mindestens 0,01 mg/kg Körpergewicht, bis höchstens 10 mg/kg Körpergewicht, vorzugsweise bis höchstens 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

### Liste der Abkürzungen:

- AIBN: α,α-Azo-bis-isobutyronitril
- Bn: Benzyl
- Brine: gesättigte wäßrige NaCl-Lösung
- CH₂Cl₂: Dichlormethan
- DCI: Desorption-Chemical Ionisation
- DIP: Diisopropylether
- DMA: Dimethylacetamid
- DME: Dimethoxyethan
- DMF: N,N-Dimethylformamid
- EE: Ethylacetat (EtOAc)
- EI: electron impact
- eq: Äquivalent
- ES: Elektrospray-Ionisation
- Et: Ethyl
- FAB: Fast Atom Bombardment
- HEP: n-Heptan
- HOAc: Essigsäure
- Me: Methyl
- MeOH: Methanol
- mp: Schmelzpunkt
- MTB: Methyltertiärbutylether
- NBS: N-Bromsuccinimid
- NMP: N-Methylpyrrolidon
- RT: Raumtemperatur
- THF: Tetrahydrofuran
- TMU: N,N,N',N'-Tetramethylharnstoff
- Tol: Toluol
- ZNS: Zentralnervensystem

### Experimenteller Teil

### Allgemeine Vorschrift zur Herstellung von Benzoyl-guanidinen (I)

### Variante A: aus Benzoesäuren (II, L = OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Mach dem Rühren über Nacht destilliert man das THF unter vermindertem Druck (Rotationsverdampfer) ab, versetzt mit Wasser, stellt mit 2 N HCl auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I)

### Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Beispiel 1: 4-(4-Fluorbenzol-N,N'-diethylsulfimidamoyl)-3-methylsulfonyl-benzoylguanidin, Dihydrochlorid

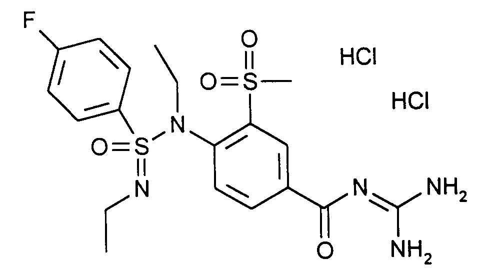

### a. 4-Fluorbenzolsulfonsäure-N,N'-diethylimidamid

146 ml einer 70% wäßrigen Ethylamin-Lösung werden bei -30°C mit 30.8 ml Brom versetzt. Man läßt auf -5°C aufwärmen, dann werden 19.2 ml 4-Fluorthiophenol zugetropft und anschließend 5 h bei RT gerührt. Danach wird die Reaktionslösung zunächst mit 100 ml einer gesättigten wäßrigen Na₂SO₃-Lösung, dann mit 200 ml einer gesättigten wäßrigen Na₂CO₃-Lösung versetzt und 5 mal mit je 200 ml EE extrahiert. Über Na₂SO₄ wird getrocknet, das Solvens wird im Vakuum entfernt, und der Rückstand wird an Kieselgel mit EE/Tol/MeOH 5:5:2 chromatographiert. Man erhält 1.8 g eines farblosen Öls.
R_{f} (EE/Tol/MeOH 5:5:2) = 0.68 MS (ES) : 231 (M+H)⁺

### b. 5-Carboxy-2-fluorbenzolsulfinsäure

15,6 g (0,124 M) Natriumsulfit werden bei 70°C in 120 ml Wasser gelöst. Unter Beibehaltung der Temperatur gibt man gleichzeitig und portionsweise 23,8 g (0,1 M) 4-Fluor-3-chlorsulfonylbenzoesäure und 10 n NaOH so zu, daß der pH zwischen 9 und 10 gehalten wird (exotherme Reaktion). Man rührt weitere 3 Stunden bei 70°C, läßt weiter 15 Minuten mit A-Kohle rühren und filtriert sodann. Das Filtrat wird unter Außenkühlung mit konzentrierter Salzsäure auf pH 0 - 1 gestellt und die kristalline 5-Carboxy-2-fluorbenzolsulfinsäure abfiltriert. Farblose Kristalle, Fp.: 167 - 170°C.

### c. 5-Carboxy-2-fluorbenzolsulfinsäure Dinatriumsalz:

erhält man durch Eintragen von 17,2 g (0,084 M) 5-Carboxy-2-fluorbenzolsulfinsäure in eine gerührte Lösung von 6,72 g (0,168 M) NaOH in einem Gemisch aus 150 ml Methanol und 30 ml Wasser: Nach Filtration von Schwebestoffen destilliert man das Lösungsmittel ab und bringt den Rückstand unter Aceton zur Kristallisation.
Farblose kristalline Substanz, Fp.: >320°C.

### d. 4-Fluor-3-methylsulfonylbenzoesäuremethylester:

Eine Suspension von 15 g (0,06 M) 5-Carboxy-2-fluorbenzolsulfinsäure Dinatriumsalz in 80 ml trockenem DMF gibt man 30 g (0,21 M) Methyliodid, rührt über 6 Stunden bei 60°C, destilliert das Lösungsmittel ab und versetzt den Rückstand mit Wasser. Man rührt 30 Min. unter Eiskühlung und filtriert den Niederschlag ab.
Farblose kristalline Substanz, Fp.: 102 - 105°C.

### e. 4-(4-Fluorbenzol-N,N'-diethylsulfimidamoyl)-3-methylsulfonylbenzoesäuremethylester

461 mg 4-Fluorbenzolsulfonsäure-N,N'-diethylimidamid und 464 mg 4-Fluor-3-trifluormethyl-benzoesäuremethylester werden in 10 ml NMP gelöst, 2.0 g Cs₂CO₃ zugegeben und 3 h bei 100°C gerührt. Nach dem Abkühlen wird das Reaktionsgemisch mit 300 ml EE verdünnt und 3 mal mit je 100 ml Wasser gewaschen. Über Na₂SO₄ wird getrocknet und der Rückstand an Kieselgel mit DIP chromatographiert. Man erhält 440 mg eines farblosen Öls.
R_{f} (DIP) = 0.27 MS (FAB) : 443 (M + H)⁺

### f. 4-(4-Fluorbenzol-N,N'-diethylsulfimidamoyl)-3-methylsulfonylbenzoylguanidin, Dihydrochlorid

180 mg 4-(4-Fluorbenzol-N,N'-diethylsulfimidamoyl)-3-methylsulfonylbenzoesäuremethylester werden nach der allgemeinen Vorschrift zur Herstellung von Benzoyl-guanidinen, Variante B umgesetzt. Man erhält 140 mg eines farblosen Öls, das mit wäßriger HCl-Lösung in das Dihydrochlorid überführt wird.
mp (Dihydrochlorid) = 193 °C (Zersetzung)
R_{f} (EE) = 0.31 MS (ES): 470 (M+H)⁺

Die Titelverbindung des Beispiels 2 wurde analog Beispiel 1 aus 4-Fluor-3-trifluormethyl-benzoesäuremethylester hergestellt:

### Beispiel 2: 4-(4-Fluorbenzol-N,N'-diethylsulfimidamoyl)-3-trifluormethyl-benzoylguanidin, Dihydrochlorid

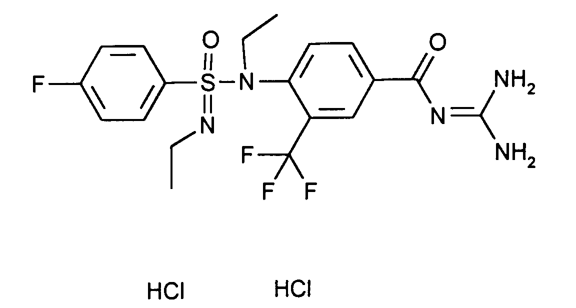

amorpher Feststoff ohne definierten Schmelzpunkt.
R_{f} (EE) = 0.51 MS (ES) : 460 (M+H)⁺

### a) 4-Fluor-3-trifluormethyl-benzoesäuremethylester

5 g 4-Fluor-3-trifluormethyl-benzoesäure und 9 ml SOCl₂ werden in 50 ml MeOH 8 h bei 60°C gerührt. Anschließend werden die flüchtigen Bestandteile im Vakuum entfernt und man erhält 5,1 g eines farblosen Öls, das ohne Reinigung weiter eingesetzt wird.
Rf (EE/MeOH 10:1) = 0.74 MS (DCI) 223 (M + H)⁺

### Beispiel 3: 3-Methylsulfonyl-4-(4-fluorbenzolsulfimidamoyl)-benzoylguanidin

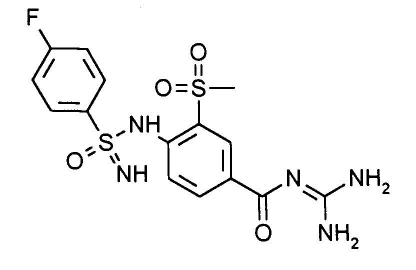

### a) 4-Fluorbenzolsulfonsäure-N,N'-bis-t-butylimidamid

100 ml t-Butylamin und 30 ml Wasser werden auf -30°C gekühlt, und bei dieser Temperatur werden 6.2 ml Brom zugetropft. 30 Minuten wird bei dieser Temperatur nachgerührt, dann wird auf -5^{o}C aufgewärmt und 4.6 g 4-Fluorthiophenol zugetropft. Man läßt auf RT erwärmen und rührt 10 h bei dieser Temperatur. Das Reaktionsgemisch wird in 200 ml gesättigter wäßriger Na₂SO₃-Lösung eingerührt, 200 ml gesättigte wäßrige Na₂CO₃-Lösung zugegeben und 30 Minuten gerührt. 4 mal wird mit je 150 ml EE extrahiert, über Na₂SO₄ getrocknet und das Solvens im Vakuum entfernt. Der Rückstand wird mit 200 ml wäßriger 1 N HCl-Lösung aufgenommen, 1 Stunde gerührt, anschließend wird mit Na₂CO₃ auf pH = 9 gestellt und 3 mal mit je 150 ml EE extrahiert. Über Na₂SO₄ wird getrocknet und das Solvens im Vakuum entfernt. Der Rückstand wird an Kieselgel mit EE/HEP 1:2 chromatographiert. Man erhält 3.2 g eines farblosen Öls.
R_{f} (DIP) = 0.40 MS(ES) :287(M+H)⁺

### b) 4-Fluorbenzolsulfonsäure-imidamid

3.1 g 4-Fluorbenzolsulfonsäure-N,N'-bis-t-butylimidamid werden in 55 ml einer 33% Lösung von HBr in Eisessig gelöst und 10 Stunden bei RT gerührt. Das Reaktionsgemisch wird langsam in 400 ml einer gesättigten wäßrigen Na₂CO₃-Lösung eingerührt und 4 mal mit 150 ml EE extrahiert. Über Na₂SO₄ wird getrocknet und das Solvens im Vakuum entfernt. Chromatographie an Kieselgel mit MTB liefert 1.4 g farbloser Kristalle,
mp 111°C.
R_{f} (EE/MeOH 10:1) = 0.44 MS (ES) : 175 (M+H)⁺

### c) 3-Methylsulfonyl-4-(4-fluorbenzolsulfimidamoyl)-benzoesäuremethylester

350 mg 4-Fluorbenzolsulfonsäure-imidamid, 460 mg 4-Fluor-3-metrylsulfonylbenzoesäuremethylester und 1.96 g Cs₂CO₃ werden in 10 ml NMP gelöst und 4 Stunden bei 100°C gerührt. Das Reaktionsgemisch wird anschließend in 100 ml einer gesättigten wäßrigen NaHCO₃-Lösung eingerührt, 3 mal mit je 100 ml EE extrahiert und die organische Phase 3 mal mit je 50 ml Wasser gewaschen. Über Na₂SO₄ wird getrocknet und das Solvens im Vakuum entfernt. Chromatographie an Kieselgel mit MTB liefert 130 mg eines farblosen Öls.
R_{f} (MTB) = 0.58 MS (ES) : 387 (M+H)⁺

### d) 3-Methylsulfonyl-4-(4-fluorbenzolsulfimidamoyl)-benzoylguanidin

120 mg 3-Methylsulfonyl-4-(4-fluorbenzolsulfimidamoyl)-benzoesäuremethylester werden nach der allgemeinen Vorschrift zur Herstellung von Benzoyl-guanidinen, Variante B, mit 90 mg Guanidin in 1 ml Isopropanol guanyliert. Reaktionsdauer 3 Stunden unter Rückfluß. Chromatographie an Kieselgel mit EE/MeOH 5 : 1 liefert 70 mg eines farblosen Öls. Schmelzpunkt des Dihydrochlorids: 240°C.
R_{f} (EE/MeOH 5:1) = 0.14 MS (ES) : 414 (M+H)⁺

### Beispiel 4: 2-Isopropyl-5-guanidinocarbonyl-benzolsulfonsäure-N,N'-dimethylimidamid

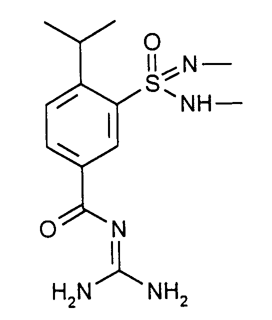

### a) 2-Isopropylbenzolsulfonsäure-N,N'-dimethylimidamid

110 ml einer 40% wäßrigen Lösung von Methylamin werden bei -30°C mit 5.7 ml Brom versetzt. 30 Minuten wird bei dieser Temperatur nachgerührt, dann wird auf -5°C erwärmt, und es werden 5.0 ml 2-Isopropylthiophenol zugetropft. 5 Stunden wird bei RT gerührt, dann wird das Reaktionsgemisch in 200 ml einer gesättigten wäßrigen Na₂SO₃-Lösung eingerührt. 200 ml einer gesättigten wäßrigen Na₂CO₃-Lösung werden zugegeben und 1 Stunde bei RT gerührt. Anschließend wird 3 mal mit je 150 ml EE extrahiert. Über Na₂SO₄ wird getrocknet und das Solvens im Vakuum entfernt. Chromatographie an Kieselgel mit EE liefert 2.8 g eines farblosen Feststoffs, mp 84-85 ^{o}C.
R_{f} (EE) = 0.48 MS (ES) : 227 (M+H)⁺

### b) 2-Isopropyl-5-iod-benzolsulfonsäure-N,N'-dimethylimidamid

2.7 g 2-Isopropylbenzolsulfonsäure-N,N'-dimethylimidamid werden in 10 ml CF₃SO₃H gelöst, dann werden 2.7 g N-Iodsuccinimid zugegeben, und es wird bei RT 4 Stunden gerührt. Das Reaktiongemisch wird langsam zu einer Mischung aus 150 ml gesättigter wäßriger NaHCO₃-Lösung und 150 ml gesättigter wäßriger Na₂CO₃-Lösung getropft. 3 mal wird mit je 150 ml EE extrahiert, über Na₂SO₄ wird getrocknet und das Solvens im Vakuum entfernt. Chromatographie an Kieselgel mit MTB liefert 3.6 g eines farblosen Öls.
R_{f} (MTB) = 0.51 MS(ES) :353(M+H)⁺

### c) 2-Isopropyl-5-n-butoxycarbonyl-benzolsulfonsäure-N,N'-dimethylimidamid

3.6 g 2-Isopropyl-5-iod-benzolsulfonsäure-N,N'-dimethylimidamid, 37.6 mg Pd(II)-acetat, 69.2 mg 1,3-Bis-(diphenylphosphino)-propan und 5.1 ml Tri-n-butylamin werden in 11 ml n-Butanol und 22 ml DMF gelöst und 6 Stunden bei 100°C gerührt. Das Reaktionsgemisch wird in 250 ml gesättigter wäßriger NaHCO₃-Lösung aufgenommen, 100 ml Wasser zugegeben und 3 mal mit je 150 ml EE extrahiert. Über Na₂SO₄ wird getrocknet und das Solvens im Vakuum entfernt.Chromatographie an Kieselgel mit EE/HEP 1 : 1 liefert 340 mg eines farblosen Öls.
R_{f} (EE/HEP 1:1) = 0.21 MS (ES) : 327 (M+H)⁺

### d) 2-Isopropyl-5-guanidinocarbonyl-benzolsulfonsäure-N,N'-dimethylimidamd

177 mg Guanidin-Hydrochlorid werden in 2.5 ml DMF gelöst und eine Lösung von 190 mg Kalium-t-butylat in 2.5 ml DMF zugegeben. 1 Stunde wird bei RT gerührt, anschließend wird eine Lösung von 110 mg 2-Isopropyl-5-n-butoxycarbonyl-benzolsulfonsäure-N,N'-dimethylimidamid in 5 ml DMF zugegeben. 24 Stunden wird bei RT gerührt, das Reaktionsgemisch in 100 ml einer gesättigten wäßrigen NaHCO₃-Lösung eingerührt, und es wird 3 mal mit je 100 ml EE extrahiert. Über Na₂SO₄ wird getrocknet und das Solvens im Vakuum entfernt. Chromatographie an Kieselgel mit EE/MeOH 1 : 8 liefert 32 mg eines weißen Feststoffs, mp 148°C (unter Zersetzung).
R_{f} (EE/MeOH 1:8) = 0.21 MS(ES):312(M+H)⁺

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2 % Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl2, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

### Inhibition des Na⁺/H⁺-Exchangers:

| Beispiel | IC₅₀ (mmol/l) |
|---|---|
| 1 | 0.68 |
| 2 | 0.053 |
| 3 | 2 |
| 4 | 6 |

## Patentansprüche

1. Sulfonimidamide der Formel I 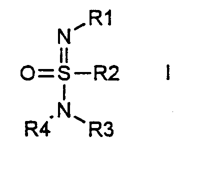 worin bedeuten:
mindestens einer der drei Substituenten R(1), R(2) und R(3)
ein Benzoylguanidin, 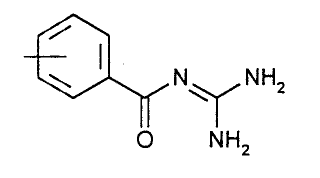
das im Phenylteil unsubstituiert oder substituiert ist mit 1 - 4 Resten ausgewählt aus der Gruppe bestehend aus Alkyl mit 1, 2, 3, 4, 5, 6, 7 oder 8 C-Atomen, Alkenyl mit 2, 3, 4, 5, 6, 7 oder 8 C-Atomen, -(CH₂)ₘ-R(14), F, Cl, Br, I,-C≡N, CF₃, R(22)SO₂-, R(23)R(24)N-CO-, R(25)-CO-, R(26)R(27)N-SO₂, -OR(35), -SR(35) oder -NR(35)R(36);
m Null, 1 oder 2;
R(14)
-(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
unabhängig voneinander Wasserstoff oder - CH₃;
R(22), R(23), R(25) und R(26)
unabhängig voneinander Alkyl mit 1, 2, 3, 4, 5, 6, 7 oder 8 C-Atomen, Alkenyl mit 2, 3, 4, 5, 6, 7 oder 8 C-Atomen, (CH₂)ₙR(29) oder -CF₃;
n Null, 1, 2, 3 oder 4;
R(29) -(C₃-C₇)-Cycloalkyl oder Phenyl,
welches nicht substituiert ist oder substituiert mit 1 - 3 Substituenten ausgewählt aus der Gruppe bestehend aus F, Cl, -CF₃, Methyl, Methoxy und -NR(30)R(31);
R(30) und R(31)
Wasserstoff oder Alkyl mit 1, 2, 3 oder 4 C-Atomen;
oder
R(23), R(25) und R(26)
Wasserstoff;
R(24) und R(27)
unabhängig voneinander Wasserstoff oder Alkyl mit 1, 2, 3 oder 4 C-Atomen;
oder
R(23) und R(24) sowie R(26) und R(27)
gemeinsam 5 oder 6 Methylengruppen, von denen eine CH₂-Gruppe durch Sauerstoff, -S-, -NH-, -NCH₃ oder -N-Benzyl ersetzt sein kann;
R(35) und R(36)
unabhängig voneinander Wasserstoff oder Alkyl mit 1, 2, 3, 4, 5 oder 6 C-Atomen;
oder
R(35) und R(36)
gemeinsam 4 - 7 Methylengruppen, von denen eine CH₂-Gruppe durch Sauerstoff, -S-, -NH-, -NCH₃ oder -N-Benzyl ersetzt sein kann;
oder
R(35)
Phenyl,
welches nicht substituiert ist oder substituiert mit 1 - 3 Substituenten ausgewählt aus der Gruppe bestehend aus F, Cl, -CF₃, Methyl, Methoxy, SO₂R(5), SO₂NR(6)R(7) und -NR(32)R(33);
R(5) Alkyl mit 1 2, 3, 4, 5 oder 6 C-Atomen
R(6) und R(7)
unabhängig voneinander Wasserstoff oder Alkyl mit 1, 2, 3 oder 4 C-Atomen;
R(32) und R(33)
unabhängig voneinander Wasserstoff oder Alkyl mit 1, 2, 3 oder 4 C-Atomen;
oder
R(35)
C₁-C₉-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
und die jeweils anderen Substituenten R(1), R(2) und R(3)
unabhängig voneinander Alkyl mit 1, 2, 3, 4, 5, 6, 7 oder 8 C-Atomen, (CH₂)ₚR(10)
p Null, 1, 2, 3 oder 4;
R(10) Phenyl,
welches nicht substituiert ist oder substituiert mit 1 - 3 Substituenten ausgewählt aus der Gruppe bestehend aus F, Cl, -CF₃, Methyl, Methoxy, -SO₂NR(17)R(8) und -SO₂R(9);
R(17) und R(8)
unabhängig voneinander Wasserstoff oder Alkyl mit 1, 2, 3 oder 4 C-Atomen;
R(9) Alkyl mit 1, 2, 3 oder 4 C-Atomen;
oder der jeweils andere Rest R( 1) und R(3)
Wasserstoff,
R(4) Wasserstoff oder Alkyl mit 1, 2, 3 oder 4 C-Atomen;
sowie deren pharmazeutisch verträgliche Salze.

2. Verbindung der Formel I nach Anspruch 1, worin bedeuten:
einer der drei Substituenten R(1), R(2) und R(3)
ein Benzoylguanidin, 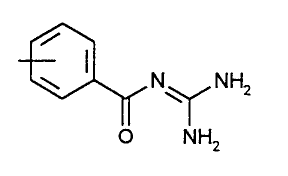
das im Phenylteil unsubstituiert oder substituiert ist mit 1 - 3 Substituenten ausgewählt aus der Gruppe bestehend aus Alkyl mit 1, 2, 3 oder 4 C-Atomen, Alkenyl mit 2, 3 oder 4 C-Atomen, -(CH₂)ₘR(14), F, Cl, CF₃, R(22)SO₂-, R(23)R(24)N-CO-, R(25)-CO-, R(26)R(27)N-SO₂, -OR(35), -SR(35) und -NR(35)R(36);
R(22) und R(25)
unabhängig voneinander Methyl oder -CF₃;
R(23), R(24), R(26) und R(27)
unabhängig voneinander Wasserstoff oder Methyl;
m Null, 1 oder 2;
R(14) -(C₃-C₆)-Cycloalkyl oder Phenyl,
welches nicht substituiert oder substituiert ist mit 1 - 2 Substituenten ausgewählt aus der Gruppe bestehend aus F und Cl, -CF₃, Methyl und Methoxy;
R(35) und R(36)
unabhängig voneinander Wasserstoff oder Alkyl mit 1, 2, 3 oder 4 C-Atomen;
oder
R(35) und R(36)
gemeinsam 4 - 6 Methylengruppen, von denen eine CH₂-Gruppe durch Sauerstoff, -S-, -NH- oder -NCH₃ ersetzt sein kann;
oder
R(35)
Phenyl,
welches nicht substituiert ist oder substituiert mit 1 - 2 Substituenten ausgewählt aus der Gruppe bestehend aus F, Cl, -CF₃, Methyl, Methoxy, SO₂R(5) und SO₂NR(6)R(7);
R(5) Alkyl mit 1, 2, 3 oder 4 C-Atomen
R(6) und R(7)
unabhängig voneinander Wasserstoff, Methyl oder Ethyl;
oder
R(35)
C₁-C₉-Heteroaryl,
das unsubstituiert oder substituiert ist mit 1 - 2 Substituenten ausgewählt aus der Gruppe bestehend aus F, Cl, CF₃, CH₃ und Methoxy;
und die jeweils anderen Substituenten R(1), R(2) und R(3)
unabhängig voneinander Alkyl mit 1, 2, 3, 4, 5 oder 6 C-Atomen oder (CH₂)ₚR(10);
p Null, 1 oder 2;
R(10) Phenyl,
welches nicht substituiert ist oder substituiert mit 1 - 2 Substituenten ausgewählt aus der Gruppe bestehend aus F, Cl, CF₃, Methyl, Methoxy, -SO₂NR(17)R(8) und -SO₂R(9);
R(17) und R(8)
unabhängig voneinander Wasserstoff, Methyl oder Ethyl;
R(9) Methyl oder Ethyl;
oder der jeweils andere Substituent R(1) und R(3)
Wasserstoff;
R(4)
Wasserstoff oder Alkyl mit 1, 2, 3 oder 4 C-Atomen.

3. Verbindung der Formel I nach Anspruch 1, in der bedeuten:
einer der Substituenten R(1), R(2) und R(3)
ein Benzoylguanidin, 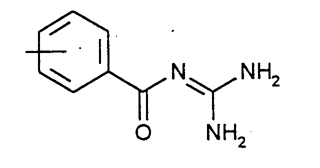
das im Phenylteil unsubstituiert oder substituiert ist mit 1 - 2 Resten ausgewählt aus der Gruppe bestehend aus Alkyl mit 1, 2, 3 oder 4 C-Atomen, R(14), F, Cl, CF₃, R(22)SO₂-, R(23)R(24)N-CO-, R(25)-CO-, R(26)R(27)N-SO₂, -OR(35), -SR(35) und -NR(35)R(36);
R(22) und R(25)
unabhängig voneinander Methyl oder CF₃;
R(23), R(24), R(26) und R(27)
unabhängig voneinander Wasserstoff oder Methyl;
R(14) -(C₃-C₆)-Cycloalkyl oder Phenyl,
welches nicht substituiert oder substituiert ist mit einem Substituenten ausgewählt aus der Gruppe bestehend aus F und Cl, -CF₃, Methyl und Methoxy;
R(35) und R(36)
unabhängig voneinander Wasserstoff oder Alkyl mit 1, 2, 3 oder 4 C-Atomen;
oder
R(35) Phenyl,
welches nicht substituiert oder substituiert ist mit einem Substituenten ausgewählt aus der Gruppe bestehend aus F, Cl, -CF₃, Methyl, Methoxy, SO₂CH₃ und SO₂NR(6)R(7);
R(6) und R(7)
unabhängig voneinander Wasserstoff, Methyl oder Ethyl;
oder
R(35) C₁ -C₉-Heteroaryl,
das unsubstituiert oder substituiert ist mit einem Substituenten ausgewählt aus der Gruppe bestehend aus F, Cl, CF₃, CH₃ und Methoxy;
und die jeweils anderen Substituenten R(1), R(2) und R(3)
unabhängig voneinander Alkyl mit 1, 2, 3 oder 4 C-Atomen oder Phenyl,
welches nicht substituiert ist oder substituiert mit einem Substituenten ausgewählt aus der Gruppe bestehend aus F, Cl, CF₃, Methyl, Methoxy, -SO₂NR(17)R(8) und -SO₂CH₃;
R(17) und R(8)
unabhängig voneinander Wasserstoff, Methyl oder Ethyl;
oder
R(1) Wasserstoff oder Alkyl mit 1, 2, 3 oder 4 C-Atomen;
oder
R(3) Wasserstoff,
R(4) Wasserstoff oder Alkyl mit 1, 2, 3 oder 4 C-Atomen.

4. Verfahren zum Herstellen einer Verbindung I nach Anspruch 1, dadurch gekennzeichnet,
daß in der Verbindung IV, in welcher mindestens einer der Substituenten R(1'*'* ), R(2'*'* ) oder R(3'*'* ), die wie R(1), R(2) und R(3) definiert sind, jedoch statt der Guanidin-Gruppe eine leicht abspaltbare Fluchtgruppe L tragen, 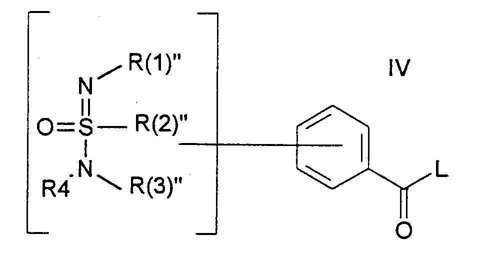 die leicht nucleophil abspaltbare Gruppe L durch Guanidin substituiert.

5. Verwendung einer Verbindung I nach Anspruch 1 zur Herstellung eines Medikaments zu Behandlung von Arrhythmien.

6. Verwendung einer Verbindung I nach Anspruch 1 zur Herstellung eines Medikaments zur Behandlung oder Prophylaxe des Herzinfarktes.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch I zur Herstellung eines Medikaments zur Behandlung von Krankheiten, bei denen die Zellproliferation eine primäre oder sekundäre Ursache darstellt.

15. Heilmittel, gekennzeichnet durch einen wirksamen Gehalt einer Verbindung der Formel I nach einem oder mehreren der Ansprüche 1 bis 3.

## Claims

1. A sulfonimidamide of the formula I 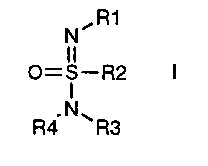 in which:
at least one of the three substituents R(1), R(2) and R(3)
is a benzoylguanidine 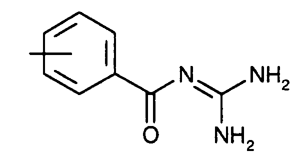
which is unsubstituted or substituted in the phenyl moiety by 1 - 4 radicals selected from the group consisting of alkyl having 1, 2, 3, 4, 5, 6, 7 or 8 carbon atoms, alkenyl having 2, 3, 4, 5, 6, 7 or 8 carbon atoms, -(CH₂)ₘ-R(14), F, Cl, Br, I, -C≡N, CF₃, R(22)SO₂-, R(23)R(24)N-CO-, R(25)-CO-, R(26)R(27)N-SO₂, -OR(35), -SR(35) or -NR(35)R(36);
m is zero, 1 or 2;
R(14) is
-(C₃-C₈)-cycloalkyl or phenyl,
which is unsubstituted or substituted by 1 - 3 substituents selected from the group consisting of F and Cl, -CF₃, methyl, methoxy and -NR(15)R(16);
R(15) and R(16)
independently of one another are hydrogen or -CH₃;
R(22), R(23), R(25) and R(26)
independently of one another are alkyl having 1, 2, 3, 4, 5, 6, 7 or 8 carbon atoms, alkenyl having 2, 3, 4, 5, 6, 7 or 8 carbon atoms, (CH₂)ₙR(29) or -CF₃;
n is zero, 1, 2, 3 or 4;
R(29) is -(C₃-C₇)-cycloalkyl or phenyl,
which is unsubstituted or substituted by 1 - 3 substituents selected from the group consisting of F, Cl, -CF₃, methyl, methoxy and -NR(30)R(31);
R(30) and R(31)
are hydrogen or alkyl having 1, 2, 3 or 4 carbon atoms;
or
R(23), R(25) and R(26)
are hydrogen;
R(24) and R(27)
independently of one another are hydrogen or alkyl having 1, 2, 3 or 4 carbon atoms;
or
R(23) and R(24) and also R(26) and R(27)
together are 5 or 6 methylene groups, of which a CH₂ group can be replaced by oxygen, -S-, -NH-, -NCH₃ or -N-benzyl;
R(35) and R(36)
independently of one another are hydrogen or alkyl having 1, 2, 3, 4, 5 or 6 carbon atoms;
or
R(35) and R(36)
together are 4 - 7 methylene groups, of which a CH₂ group can be replaced by oxygen, -S-, -NH-, -NCH₃ or -N-benzyl;
or
R(35)
is phenyl,
which is unsubstituted or substituted by 1 - 3 substituents selected from the group consisting of F, Cl, -CF₃, methyl, methoxy, SO₂R(5), SO₂NR(6)R(7) and -NR(32)R(33);
R(5) is alkyl having 1, 2, 3, 4, 5 or 6 carbon atoms
R(6) and R(7)
independently of one another are hydrogen or alkyl having 1, 2, 3 or 4 carbon atoms;
R(32) and R(33)
independently of one another are hydrogen or alkyl having 1, 2, 3 or 4 carbon atoms;
or
R(35)
is C₁-C₉-heteroaryl,
which is unsubstituted or substituted by 1 - 3 substituents selected from the group consisting of F, Cl, CF₃, CH₃, methoxy, hydroxyl, amino, methylamino and dimethylamino;
and the other substituents R(1), R(2) and R(3) in each case
independently of one another are alkyl having 1, 2, 3, 4, 5, 6, 7 or 8 carbon atoms, (CH₂)ₚR(10)
p is zero, 1, 2, 3 or 4;
R (10) is phenyl,
which is unsubstituted or substituted by 1 - 3 substituents selected from the group consisting of F, Cl, -CF₃, methyl, methoxy, -SO₂NR(17)R(8) and -SO₂R(9);
R(17) and R(8)
independently of one another are hydrogen or alkyl having 1, 2, 3 or 4 carbon atoms;
R(9) is alkyl having 1, 2, 3 or 4 carbon atoms;
or the other radical R(1) and R(3) in each case
is hydrogen,
R(4) is hydrogen or alkyl having 1, 2, 3 or 4 carbon atoms;
or its pharmaceutically tolerable salts.

2. A compound of the formula I as claimed in claim 1, in which:
one of the three substituents R(1), R(2) and R(3)
is a benzoylguanidine 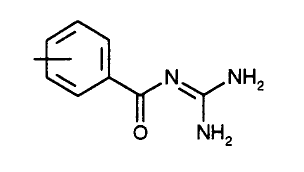
which is unsubstituted or substituted in the phenyl moiety by 1 - 3 substituents selected from the group consisting of alkyl having 1, 2, 3 or 4 carbon atoms, alkenyl having 2, 3 or 4 carbon atoms, -(CH₂)ₘR(14), F, Cl, CF₃, R(22)SO₂-, R(23)R(24)N-CO-, R(25)-CO-, R(26)R(27)N-SO₂, -OR(35), -SR(35) and -NR(35)R(36);
R(22) and R(25)
independently of one another are methyl or -CF₃;
R(23), R(24), R(26) and R(27)
independently of one another are hydrogen or methyl;
m is zero, 1 or 2;
R(14) is -(C₃-C₆)-cycloalkyl or phenyl,
which is unsubstituted or substituted by 1 - 2 substituents selected from the group consisting of F and Cl, -CF₃, methyl and methoxy;
R(35) and R(36)
independently of one another are hydrogen or alkyl having 1, 2, 3 or 4 carbon atoms;
or
R(35) and R(36)
together are 4 - 6 methylene groups, of which a CH₂ group can be replaced by oxygen, -S-,-NH- or -NCH₃;
or
R(35)
is phenyl,
which is unsubstituted or substituted by 1 - 2 substituents selected from the group consisting of F, Cl, -CF₃, methyl, methoxy, SO₂R(5) and SO₂NR(6)R(7);
R(5) is alkyl having 1, 2, 3 or 4 carbon atoms
R(6) and R(7)
independently of one another are hydrogen, methyl or ethyl;
or
R(35)
is C₁-C₉-heteroaryl,
which is unsubstituted or substituted by 1 - 2 substituents selected from the group consisting of F, Cl, CF₃, CH₃ and methoxy;
and the other substituents R(1), R(2) and R(3) in each case
independently of one another are alkyl having 1, 2, 3, 4, 5 or 6 carbon atoms or (CH₂)ₚR(10);
p is zero, 1 or 2;
R(10) is phenyl,
which is unsubstituted or substituted by 1 - 2 substituents selected from the group consisting of F, Cl, CF₃, methyl, methoxy, -SO₂NR(17)R(8) and -SO₂R(9);
R(17) and R(8)
independently of one another are hydrogen, methyl or ethyl;
R(9) is methyl or ethyl;
or the other substituent R(1) and R(3) in each case
is hydrogen;
R(4)
is hydrogen or alkyl having 1, 2, 3 or 4 carbon atoms.

3. A compound of the formula I as claimed in claim 1, in which:
one of the substituents R(1), R(2) and R(3)
is a benzoylguanidine 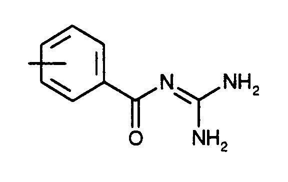
which is unsubstituted or substituted in the phenyl moiety by 1 - 2 radicals selected from the group consisting of alkyl having 1, 2, 3 or 4 carbon atoms, R(14), F, Cl, CF₃, R(22)SO₂-, R(23)R(24)N-CO-, R(25)-CO-, R(26)R(27)N-SO₂, -OR(35), -SR(35) and -NR(35)R(36);
R(22) and R(25)
independently of one another are methyl or CF₃;
R(23), R(24), R(26) and R(27)
independently of one another are hydrogen or methyl;
R(14) is -(C₃-C₆)-cycloalkyl or phenyl,
which is unsubstituted or substituted by a substituent selected from the group consisting of F and Cl, -CF₃, methyl and methoxy;
R(35) and R(36)
independently of one another are hydrogen or alkyl having 1, 2, 3 or 4 carbon atoms;
or
R(35) is phenyl,
which is unsubstituted or substituted by a substituent selected from the group consisting of F, Cl, -CF₃, methyl, methoxy, SO₂CH₃ and SO₂NR(6)R(7);
R(6) and R(7)
independently of one another are hydrogen, methyl or ethyl;
or
R(35) is C₁-C₉-heteroaryl,
which is unsubstituted or substituted by a substituent selected from the group consisting of F, Cl, CF₃, CH₃ and methoxy;
and the other substituents R(1), R(2) and R(3) in each case
independently of one another are alkyl having 1, 2, 3 or 4 carbon atoms or phenyl,
which is unsubstituted or substituted by a substituent selected from the group consisting of F, Cl, CF₃, methyl, methoxy, -SO₂NR(17)R(8) and -SO₂CH₃;
R(17) and R(8)
independently of one another are hydrogen, methyl or ethyl;
or
R(1) is hydrogen or alkyl having 1, 2, 3 or 4 carbon atoms;
or
R(3) is hydrogen,
R(4) is hydrogen or alkyl having 1, 2, 3 or 4 carbon atoms.

4. A process for preparing a compound I as claimed in claim 1, wherein in the compound IV 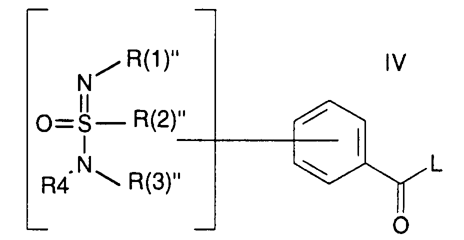 in which at least one of the substituents R(1' 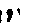 ), R(2' 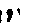 ) or R(3' 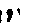 ), which are defined as R(1), R(2) and R(3), but which instead of the guanidine group carry an easily removable leaving group L,
the easily nucleophilically removable group L is substituted by guanidine.

5. The use of a compound I as claimed in claim 1 for the production of a medicament for treatment of arrhythmias.

6. The use of a compound I as claimed in claim 1 for the production of a medicament for the treatment or prophylaxis of cardiac infarct.

7. The use of a compound I as claimed in claim 1 for the production of a medicament for the treatment or prophylaxis of angina pectoris.

8. The use of a compound I as claimed in claim 1 for the production of a medicament for the treatment or prophylaxis of ischemic conditions of the heart.

9. The use of a compound I as claimed in claim 1 for the production of a medicament for the treatment or prophylaxis of ischemic conditions of the peripheral and central nervous system and of stroke.

10. The use of a compound I as claimed in claim 1 for the production of a medicament for the treatment or prophylaxis of ischemic conditions of peripheral organs and limbs.

11. The use of a compound I as claimed in claim 1 for the production of a medicament for the treatment of states of shock.

12. The use of a compound I as claimed in claim 1 for the production of a medicament for use in surgical operations and organ transplantations.

13. The use of a compound I as claimed in claim 1 for the production of a medicament for the preservation and storage of transplants for surgical measures.

14. The use of a compound I as claimed in claim 1 for the production of a medicament for the treatment of illnesses in which cell proliferation is a primary or secondary cause.

15. A pharmaceutical comprising an efficacious amount of a compound of the formula I as claimed in one or more of claims 1 to 3.

## Revendications

1. Sulfonimidamides de formule I 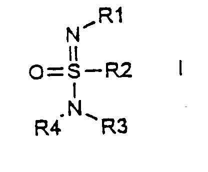 dans laquelle
au moins l'un des trois substituants R(1), R(2) et R(3)
représente une benzoylguanidine 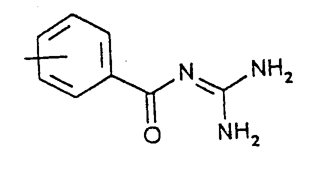
qui, sur le fragment phényle, est non substituée ou porte 1 - 4 substituants choisis dans l'ensemble constitué par des groupes alkyle ayant 1, 2, 3, 4, 5, 6, 7 ou 8 atomes de carbone, alcényle ayant 2, 3, 4, 5, 6, 7 ou 8 atomes de carbone, -(CH₂)ₘ-R(14), F, Cl, Br, I, -C≡N, CF₃, R(22)SO₂-, R(23)R(24)N-CO-, R(25)-CO-, R(26)R(27)N-SO₂, OR(35), -SR(35) et -NR(35)R(36);
m représente zéro, 1 ou 2;
R(14) représente
un groupe cycloalkyle en C₃-C₈ ou phényle,
qui est non substitué ou porte de 1 à 3 substituants choisis dans l'ensemble constitué par F et Cl, -CF₃, le groupe méthyle, le groupe méthoxy et -NR(15)R(16);
R(15) et R(16)
représentant, indépendamment l'un de l'autre, un atome d'hydrogène ou CH₃;
R(22), R(23), R(25) et R(26)
représentent, indépendamment les uns des autres, un groupe alkyle ayant 1, 2, 3, 4, 5, 6, 7 ou 8 atomes de carbone, alcényle ayant 2, 3, 4, 5, 6, 7 ou 8 atomes de carbone, (CH₂)ₙR(29) ou -CF₃;
n représente zéro, 1, 2, 3 ou 4;
R(29) représente
un groupe cycloalkyle en C₃-C₇ ou phényle,
qui est non substitué ou porte de 1 à 3 substituants choisis dans l'ensemble constitué par F, Cl, -CF₃, le groupe méthyle, le groupe méthoxy et -NR(30)R(31);
R(30) et R(31)
représentant un atome d'hydrogène ou un groupe alkyle ayant 1, 2, 3 ou 4 atomes de carbone;
ou
R(23), R(25) et R(26)
représentent des atomes d'hydrogène;
R(24) et R(27)
représentent, indépendamment l'un de l'autre, un atome d'hydrogène ou un groupe alkyle ayant 1, 2, 3 ou 4 atomes de carbone;
ou
R(23) et R(24) ainsi que R(26) et R(27)
représentent ensemble 5 ou 6 groupes méthylène, dont un groupe CH₂ peut être remplacé par un atome d'oxygène ou de soufre ou par un groupe -NH-, -NCH₃ ou -N-benzyle;
R(35) et R(36)
représentent, indépendamment l'un de l'autre, un atome d'hydrogène ou un groupe alkyle ayant 1, 2, 3, 4, 5 ou 6 atomes de carbone;
ou
R(35) et R(36)
représentent ensemble 4 - 7 groupes méthylène, dont un groupe CH₂ peut être remplacé par un atome d'oxygène ou de soufre ou par un groupe -NH-, -NCH₃ ou -N-benzyle;
ou
R(35) représente un groupe phényle
qui est non substitué ou porte de 1 à 3 substituants choisis dans l'ensemble constitué par F, Cl, -CF₃, les groupes méthyle, méthoxy, SO₂R(5), So₂NR(6)R(7) et -NR(32)R(33);
R(5) représentant un groupe alkyle ayant 1, 2, 3, 4, 5 ou 6 atomes de carbone,
R(6) et R(7)
représentant, indépendamment l'un de l'autre, un atome d'hydrogène ou un groupe alkyle ayant 1, 2, 3 ou 4 atomes de carbone;
R(32) et R(33)
représentant, indépendamment l'un de l'autre, un atome d'hydrogène ou un groupe alkyle ayant 1, 2, 3 ou 4 atomes de carbone;
ou
R(35) représente
un groupe hétéroaryle en C₁-C₉
qui est non substitué ou porte de 1 à 3 substituants choisis dans l'ensemble constitué par F, Cl, -CF₃, CH₃, les groupes méthoxy, hydroxyle, amino, méthylamino et diméthylamino;
et les autres substituants R(1), R(2) et R(3)
représentent, indépendamment les uns des autres, un groupe alkyle ayant 1, 2, 3, 4, 5, 6, 7 ou 8 atomes de carbone ou un groupe (CH₂)ₚR(10),
p représentant zéro, 1, 2, 3 ou 4;
R(10) représentant un groupe phényle
qui est non substitué ou porte de 1 à 3 substituants choisis dans l'ensemble constitué par F, Cl, -CF₃, les groupes méthyle, méthoxy, -SO₂NR(17)R(8) et -SO₂R(9);
R(17) et R(8)
représentant, indépendamment l'un de l'autre, un atome d'hydrogène ou un groupe alkyle ayant 1, 2, 3 ou 4 atomes de carbone;
R(9) représentant un groupe alkyle ayant 1, 2, 3 ou 4 atomes de carbone;
ou les autres radicaux R(1) et R(3)
représentent chacun un atome d'hydrogène,
R(4) représente un atome d'hydrogène ou un groupe alkyle ayant 1, 2, 3 ou 4 atomes de carbone;
ainsi que leurs sels pharmaceutiquement acceptables.

2. Composé de formule I selon la revendication 1, dans lequel
l'un des trois substituants R(1), R(2) et R(3)
représente une benzoylguanidine 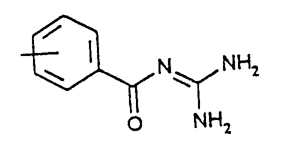
qui, sur le fragment phényle, est non substituée ou porte 1 - 4 substituants choisis dans l'ensemble constitué par des groupes alkyle ayant 1, 2, 3 ou 4 atomes de carbone, alcényle ayant 2, 3 ou 4 atomes de carbone, -(CH₂)ₘ-R(14), F, Cl, CF₃, R(22)SO₂-, R(23)R(24)N-CO-, R(25)-CO-, R(26)R(27)N-SO₂, OR(35), -SR(35) et -NR(35)R(36);
R(22) et R(25)
représentent, indépendamment l'un de l'autre, le groupe méthyle ou -CF₃;
R(23), R(24), R(26) et R(27)
représentent, indépendamment les uns des autres, un atome d'hydrogène ou le groupe méthyle;
m représente zéro, 1 ou 2;
R(14) représente
un groupe cycloalkyle en C₃-C₆ ou phényle,
qui est non substitué ou porte 1 ou 2 substituants choisis dans l'ensemble constitué par F et Cl, -CF₃, les groupes méthyle et méthoxy;
R(35) et R(36)
représentent, indépendamment l'un de l'autre, un atome d'hydrogène ou un groupe alkyle ayant 1, 2, 3 ou 4 atomes de carbone;
ou
R(35) et R(36)
représentent ensemble 4 - 6 groupes méthylène, dont un groupe CH₂ peut être remplacé par un atome d'oxygène ou de soufre ou par un groupe -NH- ou -NCH₃;
ou
R(35) représente un groupe phényle
qui est non substitué ou porte 1 ou 2 substituants choisis dans l'ensemble constitué par F, Cl, -CF₃, les groupes méthyle, méthoxy, SO₂R(5) et SO₂NR(6)R(7);
R(5) représentant un groupe alkyle ayant 1, 2, 3 ou 4 atomes de carbone,
R(6) et R(7)
représentant, indépendamment l'un de l'autre, un atome d'hydrogène ou le groupe méthyle ou éthyle;
ou
R(35) représente
un groupe hétéroaryle en C₁-C₉
qui est non substitué ou porte 1 ou 2 substituants choisis dans l'ensemble constitué par F, Cl, CF₃, CH₃ et le groupe méthoxy;
et les autres substituants R(1), R(2) et R(3)
représentent, indépendamment les uns des autres, un groupe alkyle ayant 1, 2, 3, 4, 5 ou 6 atomes de carbone ou un groupe (CH₂)ₚR(10),
p représentant zéro, 1 ou 2;
R(10) représentant un groupe phényle
qui est non substitué ou porte 1 ou 2 substituants choisis dans l'ensemble constitué par F, Cl, CF₃, les groupes méthyle, méthoxy, -SO₂NR(17)R(8) et -SO₂R(9);
R(17) et R(8)
représentant, indépendamment l'un de l'autre, un atome d'hydrogène ou le groupe méthyle ou éthyle;
R(9) représentant le groupe méthyle ou éthyle;
ou les autres radicaux R(1) et R(3)
représentent chacun un atome d'hydrogène,
R(4) représente un atome d'hydrogène ou un groupe alkyle ayant 1, 2, 3 ou 4 atomes de carbone.

3. Composé de formule I selon la revendication 1, dans lequel
l'un des substituants R(1), R(2) et R(3)
représente une benzoylguanidine 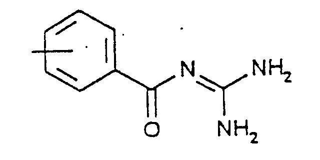
qui, sur le fragment phényle, est non substituée ou porte 1 ou 2 substituants choisis dans l'ensemble constitué par des groupes alkyle ayant 1, 2, 3 ou 4 atomes de carbone, R(14), F, Cl, CF₃, R(22)SO₂-, R(23)R(24)N-CO-,R(25)-CO-, R(26)R(27)N-SO₂, OR(35), -SR(35) et -NR(35)R(36);
R(22) et R(25)
représentent, indépendamment l'un de l'autre, le groupe méthyle ou CF₃;
R(23), R(24), R(26) et R(27)
représentent, indépendamment les uns des autres, un atome d'hydrogène ou le groupe méthyle;
R(14) représente
un groupe cycloalkyle en C₃-C₆ ou phényle,
qui est non substitué ou porte un substituant choisi dans l'ensemble constitué par F et Cl, -CF₃, les groupes méthyle et méthoxy;
R(35) et R(36)
représentent, indépendamment l'un de l'autre, un atome d'hydrogène ou un groupe alkyle ayant 1, 2, 3 ou 4 atomes de carbone;
ou
R(35) représente un groupe phényle
qui est non substitué ou porte un substituant choisi dans l'ensemble constitué par F, Cl, -CF₃, les groupes méthyle, méthoxy, SO₂CH₃ et SO₂NR(6)R(7);
R(6) et R(7)
représentant, indépendamment l'un de l'autre, un atome d'hydrogène ou le groupe méthyle ou éthyle;
ou
R(35) représente
un groupe hétéroaryle en C₁-C₉
qui est non substitué ou porte un substituant choisi dans l'ensemble constitué par F, Cl, CF₃, CH₃ et le groupe méthoxy;
et les autres substituants R(1), R(2) et R(3)
représentent, indépendamment les uns des autres, un groupe alkyle ayant 1, 2, 3 ou 4 atomes de carbone ou un groupe phényle
qui est non substitué ou porte un substituant choisi dans l'ensemble constitué par F, Cl, CF₃, les groupes méthyle, méthoxy, -SO₂NR(17)R(8) et -SO₂CH₃;
R(17) et R(8)
représentant, indépendamment l'un de l'autre, un atome d'hydrogène ou le groupe méthyle ou éthyle;
ou
R(1) représente un atome d'hydrogène ou un groupe alkyle ayant 1, 2, 3 ou 4 atomes de carbone;
ou
R(3) représente un atome d'hydrogène;
R(4) représente un atome d'hydrogène ou un groupe alkyle ayant 1, 2, 3 ou 4 atomes de carbone.

4. Procédé pour la préparation d'un composé I selon la revendication 1, caractérisé en ce que, dans le composé IV, dans lequel au moins l'un des substituants R(1''), R(2'') et R(3''), qui sont définis comme R(1), R(2) et R(3), porte au lieu du groupe guanidino un groupe partant L aisément séparable, 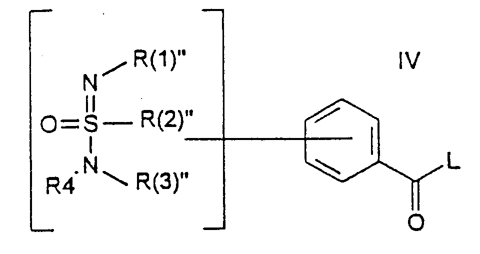 on remplace par la guanidine le groupe partant L pouvant être aisément remplacé par substitution nucléophile.

5. Utilisation d'un composé I selon la revendication 1, pour la fabrication d'un médicament destiné au traitement d'arythmies.

6. Utilisation d'un composé I selon la revendication 1, pour la fabrication d'un médicament destiné au traitement ou à la prophylaxie de l'infarctus du myocarde.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux périphérique et central et de l'apoplexie cérébrale.

10. Utilisation d'un composé I selon la revendication 1, pour la fabrication d'un médicament destiné au traitement ou à la prophylaxie d'états ischémiques d'organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des greffes d'organes.

13. Utilisation d'un composé I selon la revendication 1, pour la fabrication d'un médicament destiné à la conservation et au stockage de transplants pour des opérations chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Médicament, caractérisé par une teneur efficace en un composé de formule I selon une ou plusieurs des revendications 1 à 3.
